# EUROPEAN PATENT APPLICATION

(11) **EP 4 282 944 A1**
(43) Date of publication of application: **29.11.2023**
(21) Application number: 22175836.0
(22) Date of filing: 27.05.2022
(51) Int. Cl.: C11D 1/94, A61K 8/02, A61K 8/42, A61K 8/46, A61Q 5/02, A61Q 19/10, C11D 17/00, C11D 17/04, C11D 17/06, C11D 1/14, C11D 1/90, C11D 1/92

(54) **UNIT DOSE FOR PERSONAL CARE OR HOUSEHOLD CARE COMPOSITION**

(71) Applicant: Dalli-Werke GmbH & Co. KG, 52224 Stolberg (DE)
(72) Inventor: MÜLLER, Stefan, 50825 Köln (DE); THIEL, Rolf, 66123 Saarbrücken (DE); CHEUNG, Chi Lam, 52222 Stolberg (DE); WAGNER, Sebastian, 52531 Übach-Palenberg (DE); NICKEL, Janine, 67697 Otterberg (DE); REHFELDT, Iris, 67316 Carlsberg (DE); SCHARRER, Andreas, 68623 Lampertheim (DE)
(74) Representative: f & e patent

(57) **Abstract**

The present invention refers to powder or unit doses of personal care or household care compositions suitable for preparing a liquid or jelly ready-to-personal care or household care composition, said liquid or jelly compositions as well as the use of said tablet or unit doses for preparing said liquid or jelly compositions. The powder or unit dose detergent composition comprises (i) from 1 to 40 wt.-%, based on the total weight of the powder or unit dose, of at least one amphoteric surfactant (betaines or sulphobetaines), (ii) at least one anionic surfactant (alkyl sulphates or sulphonates), and (iii) less than 5 wt-% of a polymeric thickening agent, selected from non-charged polysaccharides and polymers comprising acrylic monomers. A unit dose, represented by a sachet, package or pouch containing a pre-dosed amount of the powder, or a tablet prepared of a pre-dosed amount of the composition is also disclosed, as well as a kit for preparing the liquid or jelly personal care or household care detergent composition comprising the powder or unit dose and a container having an opening shaped and sized to feed said tablet or unit dose through said opening and a cap or head for closing said opening of the container.

## Description

The present invention relates to powder or unit doses comprising an amphoteric and anionic surfactant suitable for preparing a liquid or jelly composition, in particular a personal care or household care composition, to a liquid or jelly composition prepared by dissolving said powder or unit dose in water and to the use of such powder or unit dose for preparing said liquid or jelly composition.

For laundry or automatic dishwashing powdered detergent compositions or detergent compositions in unit portions have become increasingly popular as they are easy to handle and avoid over- or under-dosing and spillage of detergent. For other applications, like cleaning hard surfaces e.g., in bath, kitchen, floor or even for cosmetic, body or hair care applications, like e.g., shampoo, shower or bath compositions, hand or body wash compositions, liquid cleaning compositions are clearly more suitable. Accordingly, most of these cleaners are offered as aqueous liquid cleaning compositions comprising water as the main ingredient. Thus, using said compositions involves carrying and transporting significant amounts of water as well as using a huge number of mostly "one-way" plastic containers.

Therefore, in the present application it is discussed to provide detergent compositions in form of powder or unit doses provided in suitable portions, e.g., as tablets, pouches or sachets, for preparing such cleaning compositions "ready-for-use" by the consumers themselves. This can be done by adding said powder or unit dose to a predetermined amount of water.

Personal care or household care compositions, which are intended to be offered as powders/unit portions have to be carefully balanced in view of storage stability, dissolution properties and performance and further to aesthetic and acceptance aspects. The compositions must dissolve easily, have a high cleaning power while still producing a stable high-quality foam and should not irritate the skin. Different approaches to solve these hurdles are disclosed in the literature.

DE 10 2017 206 013 A1 describes a washing or cleaning agent with improved foaming properties using betaines to reduce the amount of linear or branched alkyl benzyl sulfonate.

EP 2 011 545 B1 describes structured compositions comprising betaine, an anionic surfactant and a branched fatty alcohol for use in personal care products.

US 5,840,676 describes a microemulsion light duty detergent with desirable cleansing properties and mildness to the skin comprising a nonionic surfactant, ethoxylated alkyl ether sulfate, a sulfonate or sulfonate anionic surfactant, betaine, and optionally an alkyl monoalkanol amide.

EP 3 825 392 describes detergent tablets comprising an effervescent system suitable for preparing a liquid ready-to-use detergent or cleaning composition, based on an effervescent system, a non-ionic surfactant and a chelating agent.

DE 42 24 714 A1 describes a detergent comprising quaternized fatty acid alkanolamine esters, anionic surfactants and a betain that results in a foaming detergent with high stability.

WO-A-9920727 discloses a cleaning composition for hard surfaces containing multiply-substituted protease variants in combination with a surfactant from the group of amphoteric and/or anionic and/or non-ionic surfactants.

EP 0 967 957 B1 discloses a body care formulation including surfactants, initiators for phase separation and water.

EP 3 724 309 B1 refers to a liquid cleaning composition for pre-treatment of fabrics comprising alkoxylated anionic, non-ionic and amphoteric surfactants, a water miscible solvent and water.

WO-A-0008129 discloses a laundry detergent composition for washing fabrics by hand containing a high-foaming anionic surfactant and at least one milder cosurfactant like amphoteric surfactants.

US 6,506,717 B1 describes a dishwashing composition comprising modified alkylbenzene sulfonates and different cosurfactants like betaines or amine oxides.

DE 101 26 706 B4 describes a solid hand dishwashing composition with a high amount of surfactants including at least one anionic and on amphoteric surfactant.

The object of the present invention is to provide a personal care or household care composition in dry, pre-portioned, compact, weight and space saving form allowing to prepare a liquid or jelly composition by the consumers themselves in an easy and plastic waste avoiding manner. The liquid or jelly composition should be preparable fast, non-elaborately and without any solid, greasy or unattractive remainders in the liquid composition. The resulting composition should produce a nice and stable foam with a high cleaning efficiency but without irritating the skin.

This object is met by the composition for personal care or household care in form of a powder or unit dose for preparing a liquid or jelly detergent according to the invention. The powder or unit dose for preparing the personal care or household care compositions according to the present invention comprises an amphoteric surfactant and an anionic surfactant while using less than 5 wt.-% of thickening agent. A composition combining an amphoteric with an anionic surfactant produces a stable foam with exceptional cleaning performance while giving almost no skin irritation. It is well known that high sudsing anionic surfactants with good foam volume may give high skin irritation. Amphoteric cosurfactants can synergistically reduce the skin irritation, thus providing a mild cleaning composition. Due to the combination of surfactants the liquid or jelly personal care or household care composition has a high viscosity suitable for use, allowing the addition of less than 5 wt.-% thickener. Normally, thickening agents may suppress foam volume as well as foaming speed when added to a certain amount to achieve required viscosity. Compositions with a high amount of polymeric thickener tend to dissolve very slowly and such thickeners might turn the liquid composition from transparent to cloudy. Additionally, the use of said thickeners can provide an unpleasant consistency. Further, by not using synthetic polymers as thickeners, environmental problems are reduced.

According to the present invention it has been found that a detergent personal care or household care composition in form of a powder or unit dose comprising said combination of surfactants can be suitably stored and readily dissolved in water resulting in a transparent liquid or jelly composition suitably foaming for comfortable use. The stable foam with a high cleaning performance can be used for hard surfaces as well as cosmetic applications by balancing the ingredients of the composition for the intended purpose.

Here, a material or composition is termed "transparent" when at least 85 % of the light of the visible spectrum can transmit through the material or composition, preferably at least 90 % or at least 92 % or at least 95 % of the visible light.

The term "detergent composition" describes a composition with a cleaning effect, which according to the invention is preferably a detergent composition coming into contact with human skin during handling. Examples for household care compositions are,but are not limited to hand dishwashing or hand laundry compositions, bath or floor cleaning compositions, examples for personal care compositions are, but are not limited to shampoo, hair conditioner, shower gel, hand cleansing composition, face cleansing composition, cleansing composition for the genital area, body scrub composition or bath additives.

The terms "comprising", "comprises" and "comprised of" as used herein are synonymous with "including", "includes" or "containing", "contains", and are inclusive and open-ended and do not exclude additional, non-recited members, elements or method steps. It will be appreciated that the terms "comprising", "comprises" and "comprised of" as used herein comprise the terms "consisting of", "consists" and "consists of".

### Amphoteric surfactant

Amphoteric or ampholytic surfactants have several functional groups that can ionize in aqueous solution, giving the compounds anionic or cationic character, depending on the conditions of the medium. Near the isoelectric point, amphotensides form internal salts, which can make them sparingly soluble or insoluble in water. Amphotensides are divided into ampholytes and betaines, the latter being in solution as zwitterions. Ampholytes are amphoteric electrolytes, i.e., compounds that have both acidic and basic hydrophilic groups and thus behave acidic or basic depending on the condition.

Preferred amphoteric surfactants described herein are betaines and its derivatives, like alkylamidoalkyl betaines, preferably linear C₈ - to C₁₆-alkylamidopropyl betaines, e.g. caprylamidopropylbetaine (N-(3-octanoyl)aminopropyl)-N- carboxymethyl-N,N-dimethyl-propanaminium) or capramidopropylbetaine (N-(3-decanoyl)aminopropyl)-N-carboxymethyl-N,N-dimethyl-propanaminium), or, most preferably, cocoamidopropyl betaine.

Further preferred amphoteric surfactants are sulfobetaines e.g., such represented by but not limited to coco dimethyl sulfopropyl betaine, stearyl dimethyl sulfopropyl betaine, cocoamidopropyl hydroxysultaine and lauryl dimethyl sulfoethyl betaine or lauryl bis- (2-hydroxyethyl) sulfopropyl betaine.

Further preferred amphoteric surfactants are e.g., alkyl amine oxides, in particular amine oxides having one long alkyl chain including 7, 8, 9, 10, 11, 12, 13, 14, 15, 16, 17, 18, 19, 20, 21, 22, 23 or 24 carbon atoms, and two short alkyl chains including 1, 2 or 3 carbon atoms. Even more preferred are C₁₀₋₁₈ alkyl dimethyl amine oxides, particularly preferred is N-dodecyl-N,N-dimethyl amine oxide.

Further preferred amphoteric surfactants belong to the group of amphoacetates and amphodiacetates, like but not limited to cocoamphodiacetate, lauroamphodiacetate, caprylamphodiacetate and capryloamphodiacetate.

For the mentioned amphoteric surfactants any counter-ions are suitable, like e.g., ammonium, potassium, monoethanolamine (MEA) or triethanolamine (TEA), and/or chloride, hydroxy, carbonate, bicarbonate or acetate. Preferred counter-ions are Na⁺, MEA and/or Cl⁻.

The amphoteric surfactants can be present in the composition of the invention in a total amount of from 1 to 40 wt.-%, preferably in an amount of from 3 to 35 wt.-%, even more preferred in an amount of from 5 to 30 wt.-%. This definition should be considered as defining that each of the mentioned amphoteric surfactants can be individually present in the composition in the defined range, however, the total amount of these types of surfactants is also in said range.

### Anionic surfactant

Besides the mentioned amphoteric surfactant, the composition comprises at least one anionic surfactant.

Preferred anionic surfactants comprise or consist of a hydrophobic chain CₙH₂ₙ₊₁ with n = 6 to 24, preferably 8 to 20, more preferred 8 to 18, and an anionic hydrophilic group. The hydrophilic group can e.g., be a carboxylate (CₙH₂ₙ₊₁COO⁻X), a sulphate (CₙH₂ₙ₊₁OSO³⁻ X), a sulphonate (CₙH₂ₙ₊₁SO³⁻ X), a phosphate (CₙH₂ₙ₊₁OPO(OH)O⁻ X), an anionic amino acid, a (sulpho)succinate, a (sulfo)acetate, an isethionate, a taurate or any other anionic hydrophilic group. The counter-ion (X) can be any suitable counter-ion, like e.g., Na⁺, K⁺, NH₄⁺, MEA or TEA.

Surfactants of the alk(en)yl sulphates type, sulphonates, alkoxylated alk(en)yl sulphates, ester sulphonates and/or soaps are preferably used as anionic surfactants. The preferred alk(en)yl sulphates are the alkali metal salts and in particular the sodium salts of the sulphuric acid half-esters of the C₈-C₁₈ fatty alcohols, for example from coconut fatty alcohol, tallow fatty alcohol, lauryl, myristyl, cetyl or stearyl alcohol or of the C₈-C₂₀ oxoalcohols and those half-esters of secondary alcohols of this chain length. Particularly preferred anionic surfactants are lauryl sulfate and/or coco sulfate, even more preferred the sodium salts thereof, sodium lauryl sulfate and/or sodium coco sulfate. For usage in household care compositions lauryl sulfate might be preferred, whereas for personal care compositions coco sulfate might be used as a preferred surfactant, however, both types are suitable for both applications.

Common types of sulphonate surfactants are alkyl- or alkyl aryl sulphonates (e.g., sodium C₁₄₋₁₆ alkyl sulfonate, naphthalene sulphonate, alkyl naphthalene sulphonate), paraffin sulphonates, alkyl benzene sulphonate (ABS) or alpha-olefin sulphonates. However, preferably the personal care or household care composition is free of ABS as anionic surfactant.

The properties of sulfate or sulphonate surfactants can optionally be modified by introducing ethylene and/or propylene oxide units in the chain, preferably ethylene oxide units.

The ethoxylated alkyl ether sulfate may be made by sulfating the condensation product of ethylene oxide and C₈-₁₈ alcohols, and neutralizing the resultant product. The ethoxylated alkyl ether sulfates differ from one another in the number of carbon atoms in the alcohols and in the number of moles of ethylene oxide reacted with one mole of such alcohol. The C₈-₁₈ ethoxylated alkyl ether sulfate surfactants preferably have 1 to 22 ethylene oxide (EO) units and the counterion is an ammonium or metal cation, most preferably sodium. Preferred ethoxylated alkyl ether sulfates contain 8 to 18, like 10 to 16 carbon atoms in the fatty alcohol and 3 to 10 ethoxy groups, e.g., sodium myristyl (3 - 6 EO) sulfate or sodium laureth (2 - 7 EO) sulfate.

Ethoxylated C₈-₁₈ alkylphenyl ether sulfates containing from 2 to 6 EO units in the molecule are also suitable for use according to the invention. These detergents can be prepared by reacting an alkyl phenol with 2 to 6 moles of ethylene oxide, sulfating and neutralizing the resultant ethoxylated alkylphenol.

Further preferred types of anionic surfactants are sulfosuccinates (as mono- or di-esters or a mixture thereof) and sulfoacetates, as well as isethionates, due to their mild cleaning performance. Examples of suitable anionic surfactants are C₆₋₂₀ alkyl sulfosuccinate, C₆₋₂₀ alkyl sulfoacetate or C₆₋₂₀ alkyl isethionate, in particular as sodium, potassium or ammonium salts.

If suitable, the sulfosuccinate or sulfoacetate surfactant can also be ethoxylated, e.g., by 3 to 12 EO units. Examples of suitable sulfosuccinates are obtainable under the tradename KLK Tensomild H026 by KLK Oreo and possible sulfoacetates are sodium lauryl sulfoacetate (e.g., Stepan Lathanol LAL available by Stepan) or disodium laureth sulfoacetate, or a mixture of those two. Such a mixture is available by Stepan under the tradename Stepan Mild LSB.

Preferred alkyl phosphates and alkyl ether phosphates can be made by treating fatty alcohols or alcohol ethoxylates with a phosphorylating agent which yields in a mixture of mono- and di-esters of phosphoric acid.

Further preferred anionic surfactants are amino acid surfactants. Amino acid surfactants can be derived from carboxylate salt of amino acids, wherein the amine group situated on the α-carbon or β-carbon of an amino acid salt is acylated with a C₈ to C₂₂ fatty acid derivative.

Examples of the amino acid surfactants are salts of alanine, arginine, aspartic acid, glutamic acid, glycine, isoleucine, lysine, phenylalanine, serine, tyrosine, valine, sarcosine and any mixture thereof. More preferred are amino acid surfactants such as sodium or potassium salts of caproyl glutamate, cocoyl glutamate, lauroyl glutamate, stearoyl glutamate, myristoyl glutamate, caproyl methyl β-alaninate, cocoyl methyl β-alaninate, lauroyl methyl β-alaninate, stearoyl methyl β-alaninate, myristoyl methyl β-alaninate, caproyl β-alaninate, cocoyl β-alaninate, lauroyl β-alaninate, stearoyl β-alaninate, myristoyl β-alaninate, caproyl glycinate, cocoyl glycinate, lauroyl glycinate, stearoyl glycinate, myristoyl glycinate, caproyl sarcosinate, cocoyl sarcosinate, lauroyl sarcosinate, stearoyl sarcosinate, myristoyl sarcosinate, caproyl aspartate, cocoyl aspartate, lauroyl aspartate, stearoyl aspartate, myristoyl aspartate, and mixtures thereof.

Especially preferred amino acid surfactants in the personal care or household care composition are selected from sodium lauroyl sarcosinate, sodium cocoyl glycinate, sodium cocoyl glutamate, sodium lauroyl glutamate, or a mixture thereof. Most preferred is sodium lauroyl glycinate.

The anionic surfactant(s) is/are be present in the composition of the invention in a total amount of from 5 to 60 wt.-%, preferably in an amount of from 10 to 55 wt.-%, more preferred from 13 to 52 wt.-%, even more preferred from 15 to 50 wt.-%. This definition should be considered as defining that each of the mentioned anionic surfactants can be individually present in the composition in the defined range, however, the total amount of these types of surfactants is also in said range.

Preferably, the weight ratio of the total amount of the amphoteric surfactant(s) to the total amount of anionic surfactant(s) in the detergent or cleaning composition is from 4:1 to 1:12,5, preferably from 3:1 to 1:10, in particular from 2:1 to 1:8, especially from 1:1 to 1:5 (wt/wt), calculated on basis of their salts having the same counter-ion.

### Polymeric thickening agent

The composition as provided herewith allows the preparation of a liquid or jelly detergent composition, in particular aqueous personal care or household care composition. According to the present application the liquid or jelly composition has a viscosity at 20 C of 0.5 to 10⁵ Pa s, preferably of 1 to 10⁴ Pa s, wherein "liquid" is more in the range of 0.5 to 1000 Pa s, and "jelly" is more in the range above 1000 Pa s. In any case the liquid or jelly composition is pourable or can be squeezed through an opening of a squeezable bottle like e.g., a hand dishwashing detergent, a shampoo or shower gel flask at a temperature above 0°C, preferably above 10°C, like in the temperature range of from 15 to 80° C.

The ready-prepared liquid or jelly composition has a pleasant high viscosity through dissolution of the ingredients, in particular the solid surfactants; especially the presence of amphoteric surfactant(s) render(s) further thickening agents unnecessary.

In a preferred embodiment, the personal care or household care composition comprises less than 5 wt.-% of polymeric thickener, more preferred less than 4 wt.-%, even more preferred less than 3 wt.-%. Most preferred the composition is free of any polymeric thickener.

In the present application, the term "polymeric thickener" describes compounds which can increase the viscosity of the liquid or jelly composition like non-charged polysaccharides, e.g., starch and its derivatives, guar gum or xanthan gum. Another class of polymeric thickeners are synthetic polymers like polyacrylates (referring to any polymer comprising (meth)acrylate as monomeric units). It is particularly preferred that the composition does not comprise any acrylic polymers or copolymers. However, the polymeric thickeners mentioned herein should be considered as examples, not as limiting the invention to said specific thickeners. If a water-soluble polymer, e.g., a starch polymer is used as a water-soluble package material for providing a unit-dose, said packaging polymer is provided as a film or sheet resulting also in an amount below 5 wt.-% of the total weight of the whole unit-dose package.

Neither the surfactants or builders disclosed in the application nor inorganic salts like sodium chloride or sodium sulfate belong to the category of "thickener" according to the present invention. Therefore, the viscosity can optionally be regulated by the addition of a salt, e.g., sodium chloride or sodium sulfate.

Further, the conditioning polymers, described below as additional ingredients, in particular for the inclusion in personal care compositions, are not falling under the definition of the polymeric thickeners according to the present disclosure.

### Builder system

Optionally, the present invention also comprises a builder system. The main functions of builder systems are to soften the washing water, to provide a buffering capacity to the washing liquid and to have an anti-redeposition or dispersing function. The physical properties of the detergent composition are also depending on the builders that are used. Acrylates as well as inorganic phosphates preferably are excluded from the composition due to their negative environmental impact.

Builders can generally be added to the composition in acid form, neutralized or in a partly neutralized form. When used in a partly or completely neutralized form alkali metal salts are preferred, like sodium, potassium and lithium or ammonium salts.

Inorganic non-phosphate builders include, but are not limited to, phosphonates, silicates, carbonates, sulphates, and aluminosilicates.

Organic builders include, but are not limited to, a wide variety of (poly)carboxylated compounds having one or more carboxylate groups, e.g., citric acid, adipic acid, succinic acid, glutaric acid, malic acid, tartaric acid, maleic acid, fumaric acid, sugar acids, aminocarboxylic acids, nitrilotriacetic acid (NTA). Preferred salts are the salts of polycarboxylic acids such as citric acid, adipic acid, succinic acid, glutaric acid, tartaric acid, sugar acids and mixtures thereof. Preferably the composition comprises sodium citrate and/or citric acid.

Further suitable builder substances are polyacetals, which can be obtained by reacting dialdehydes with polyol carboxylic acids having 5 to 7 C atoms and at least 3 hydroxyl groups, for example as described in European patent application EP-A-0 280 223. Preferred polyacetals are obtained from dialdehydes such as glyoxal, glutaraldehyde, terephthalaldehyde as well as mixtures thereof and from polyolcarboxylic acids such as gluconic acid and/or glucoheptonic acid.

Further suitable organic builder substances are dextrins, for example oligomers or polymers of carbohydrates, which can be obtained by partial hydrolysis of starches. Preferably, the hydrolysis products have average molecular weights in the range of 400 to 500000 g/mol. A polysaccharide with a dextrose equivalent (DE) in the range of 0.5 to 40, in particular of 2 to 30, is preferred, DE being a common measure of the reducing effect of a polysaccharide compared to dextrose, which has a DE of 100.

The builder system can comprise one or several of the mentioned builder compounds. According to the invention the builder system is preferably present in a total amount ranging from 10 to 80 wt.-%, e.g., from 15 to 60 wt.-%, preferably in a total amount ranging from 20 to 55 wt.-%, more preferably in a total amount ranging from 22 to 53 wt.-%, even more preferred from 25 to 50 wt.-% based on the total weight of the composition of the claimed invention. Thus, either of the builder compounds can be present in an amount as defined here, but in any case, the total amount of the builder compounds mentioned here is also within the defined range.

In a preferred embodiment, the builder system consists of two ingredients wherein one component is an acid, e.g., citric acid. If the builder system of the claimed composition comprises two ingredients wherein one component is an acid, the ratio of the acidic to the other component preferably ranges from 1:10 to 4:1, more preferably from 1:6 to 3:1, even more preferably from 1:5 to 2:1, most preferably from 1:4 to 1:1 (wt/wt). Most preferably, the builder system according to the invention comprises at least citric acid and sodium citrate. It is particularly preferred that citrate/citric acid together represent at least 60 wt.-% of the builder system, more preferred at least 80 wt.-%, even more preferred at least 90 wt.-% of the builder system. In an embodiment the builder system might consist solely of citrate and citric acid.

### Co-builders

In addition, co-builders can be used to increase the efficiency of the builder system. The most preferred co-builders are complexing agents further described below.

Further useful organic co-builders are, e.g., acetylated hydroxycarboxylic acids or salts thereof, which may optionally also be present in lactone form and which contain at least 4 carbon atoms and at least one hydroxyl group and a maximum of two acid groups. Such co-builders are described, for example, in international patent application WO-A-95/20029.

A further class of substances with co-builder properties are the phosphonates. These are in particular hydroxyalkane or aminoalkane phosphonates. Among the hydroxyalkane phosphonates, the 1-hydroxyethane-1,1 -diphosphonate (HEDP) is of particular importance as a co-builder. It is preferably used as a sodium salt, with the disodium salt reacting neutrally and the tetrasodium salt alkaline (pH 9). Preferred aminoalkane phosphonates are ethylenediaminetetramethylene phosphonate (EDTMP), diethylenetriaminepentamethylene phosphonate (DTPMP) and their higher homologues. They are preferably used in the form of the neutrally reacting sodium salts, e.g., as hexasodium salt of EDTMP or as hepta- and octa-sodium salt of DTPMP. The preferred co-builder from the phosphonate class is HEDP. The aminoalkane phosphonates also have a pronounced heavy metal binding capacity. Accordingly, it may be preferable, especially if the agents also contain bleach, to use aminoalkane phosphonates, in particular DTPMP, or to use mixtures of the phosphonates mentioned. In addition, all compounds capable of forming complexes with alkaline earth ions can be used as co-builders.

Typically, the powder or unit dose composition according to the present invention comprise a total amount of from 0.1 up to about 30 wt.-%, preferably from 0.3 to 25 wt.-%, more preferably from 0.5 to 20 wt.-%, even more preferred from 0.9 to 17 wt.-% or of 1.5 to 15 wt.-% of a co-builder based on the total weight amount of the tablet or unit dose composition. The composition, according to the present invention, may comprise any of said co-builder individually or a mixture thereof.

### Powder or unit dose composition

To avoid additional drying steps during manufacturing of the powder for personal or household care applications solid raw materials are preferred. Amphoteric and anionic surfactants in powder form are particularly preferred as raw materials.

The powder or unit dose composition of the present invention is intended to provide a ready-to-use liquid or jelly personal care or household care composition by just adding a specific amount of powder or a single unit dose to a predetermined amount of water. The powder or unit dose is therefore either provided in a defined weight amount, calculated to be dissolved in a defined volume of water, or a dosing device is provided allowing to dose the powder correctly. To allow a pleasant handling for preparing the ready-to-use liquid or jelly composition, the weight amount of the powder preferably is calculated for a water volume in the range of 100 ml to 1 I, e.g., 100 ml, 150 ml, 200 ml, 250 ml, 300 ml, 400 ml, 500 ml, 600 ml, 700 ml, 750 ml, 800 ml or 1 I. The powder or unit dose for providing the liquid or jelly composition in this volume preferably is provided in a weight amount of 10 to 40 g, calculated such that preferably an amount of 20 to 30 g, more preferred 24 to 26 g is added to 225 ml of water. However, the amounts mentioned herein should be considered as preferred examples, not as limiting the invention to said specific amounts.

The powder can be provided e.g., in form of ready-dosed packages, e.g., paper bags or small paper boxes comprising a given amount of the powder (i.e. a unit dose), or together with a dosing device, like e.g., a measuring cup or something similar. Further, the unit dose can be provided as a tablet, a sachet, a pouch or any other suitable readily usable pre-dosed unit. The pre-dosed unit can be comprised in a water-soluble material, e.g., a water-soluble polymer, preferably a bio-degradable polymeric material, e.g., starch, polyvinyl alcohol or poly lactic acid, without being limited to the mentioned.

The liquid or jelly personal care or household care composition obtained by adding a specific amount of powder / unit dose to water should not comprise any solid remainder(s), preferably no greasy/oily film on the surface and should be pleasantly to prepare and to use. Therefore, it is particularly preferred that the composition comprises only ingredients fully dissolvable in water in the amounts as calculated for the powder / unit dose ingredients in the water volume as specified. Most preferred is a composition without an effervescent system.

The term "effervescent system" relates to a combination of compounds or mixtures of compounds which result in the generation and release of a gas when administered to a liquid or when in contact with a liquid. Preferably, the term "effervescent system" refers to a system comprising at least one acidic and at least one basic component, preferably components capable of reacting to form a gaseous species, e.g., carbon dioxide or oxygen, upon contact with a liquid, e.g., water. Examples of such systems are disclosed in DE 2 132862, EP 0 286 085 and US 2004/0116317 A1.

With "fully dissolvable" is meant that the ingredient(s) dissolve in the water volume as specified for the powder / unit dose at 30°C within 10 to 15 min without any remainder due to slewing (not shaking) and wherein all the ingredients remain dissolved during storage for at least 12 weeks.

The powder or unit dose composition of the present invention when solved in water preferably provides an acidic to neutral pH (pH 3 to pH 7, preferably pH 3.5 to pH 6) due to the components of the composition. This pH is obtained by applying a unit amount of the detergent composition to water in a ratio 1:8 to 1:12 (detergent:water), preferably in a ratio of about 1:10 (detergent:water). This can be obtained by adding an amount of e.g. 24-26 g (e.g., 25 g) of the powder into 225 ml water at 30 to 40 °C and measuring the pH after all ingredients are solved and after cooling to 20 to 25 °C.

The powder or unit dose composition according to the invention preferably comprises a water content ranging from 0 to 5, preferably from 0 to 4, more preferably from 0 to 3, even more preferably from 0 to 2, still even more preferably from 0 to 1, still even more preferably from 0 to 0.5, and most preferably from 0 to 0.25 wt.-% of the total composition. The water content is defined by the amount of water which evaporates when the composition or components is/are heated to 105 °C.

### Further ingredients of personal care or household care composition

Preferably the personal care or household care composition according to the invention comprises besides the amphoteric and the anionic surfactant(s) as cited above at least one or more further component(s) selected from: chelating agent(s), further surfactants, pH adjusting ingredients, colorants, dyes, preservatives, perfumes, natural oils and fats, mineral salts, and optionally conditioning agents. More preferably the composition comprises at least one component selected from: chelating agents, further surfactants, pH adjusting agents and preservatives; most preferably the composition comprises at least a chelating agent and/or at least one preservative.

Said one or more additional component(s) amount(s) to a total amount of from 2 to 50 wt.-% based on the total weight of the tablet or unit dose composition, preferably from 5 to 45 wt.-%, more preferred from 7 to 40 wt.-%, even more preferred from 8 to 35 wt.-% of the total weight of the composition.

### Chelating agent

The composition of the present invention preferably contains a chelating agent. The chelating agent(s) have the double-function of chelating ions in the solution to prevent any precipitation in the liquid or jelly composition, and further to support the cleaning result of the composition. Chelating agents can include any of those known in the art, however, according to the invention are different from citric acid or citrate. Chelating agents can be used as co-builder to complement the builder system.

Preferred chelating agents are biodegradable chelating agents like amino acid-based chelating agents, in particular methyl glycine di-acetic acid (MGDA) and di-carboxymethyl glutamic acid (GLDA), preferably as alkali metal, or alkaline earth, ammonium or substitutes ammonium salts thereof, in particular as sodium salts. Another preferred biodegradable chelating agent for use herein is ethylene diamine N,N'- disuccinic acid, or alkali metal, or alkaline earth, ammonium or substitutes ammonium salts thereof or mixtures thereof. Further carboxylate chelating agents include tartaric acid, oxalic acid, salicylic acid, aspartic acid, glutamic acid, glycine, malonic acid, lactic acid or mixtures thereof.

Ethylenediamine N,N'- disuccinic acids, especially the (S,S) isomer have been extensively described in US patent 4, 704, 233. Ethylenediamine N,N'- disuccinic acids is, for instance, commercially available under the tradename ssEDDS^{®} from Palmer Research Laboratories. Further amino carboxylates that may be used herein include ethylene diamine terra acetates, diethylene triamine pentaacetates, diethylene triamine pentaacetate (DTPA),N- hydroxyethylethylenediamine triacetates, nitrilotri-acetates, ethylenediamine tetrapropionates, triethylenetetraaminehexa-acetates, ethanol-diglycines, propylene diamine tetracetic acid (PDTA), both in their acid form, or in their alkali metal, ammonium, and substituted ammonium salt forms.

Suitable phosphonate chelating agents are diethylene triamine penta methylene phosphonate (DTPMP) and ethane 1-hydroxy diphosphonate (HEDP). Such phosphonate chelating agents are commercially available from Monsanto under the trade name DEQUEST^{®}.

Further chelating agents are ethylenediamine-N,N'-tetraacetic acid (EDTA) or ethylenediamine-N,N'-disuccinic acid (EDDS) or the alkali metal, alkaline earth metal, ammonium, or substituted ammonium salts thereof, or mixtures thereof. Preferred EDTA or EDDS compounds are the free acid form and the sodium salt or complex thereof.

Still further chelating agents are iminodiacetic acid derivatives such as 2-hydroxyethyl diacetic acid or glyceryl imino diacetic acid, described in EP 0317542 A2 and EP 0399133 A1.

Typically, the powder or unit dose compositions according to the present invention comprise a total amount of from 0.1 up to about 15 wt.-%, preferably from 0.3 to 10 wt.-%, more preferably from 0.5 to 8 wt.-%, even more preferred from 0.9 to 7 wt.-% or 1.5 to 6 wt.-% of a chelating agent, or a mixture of chelating agents (different from citric acid or citrate) based on the total weight amount of the tablet or unit dose composition. Preferably the compositions comprise only biodegradable chelating agents, most preferred selected from MGDA and GLDA, or a combination of those.

### Additional Surfactants

Besides the amphoteric and the anionic surfactants mentioned above as necessary ingredients, the personal care or household care composition may comprise additionally a total amount of from 0.1 to 50 wt.-%, preferably 0.3 to 48 wt.-%, 0.5 to 45 wt.-%, 1 to 43 wt.-% or 1.5 to 40 wt.-% of any further surfactant(s). The mentioned ranges refer to the total amount of the added additional surfactants, thus, if a mixture of surfactants is comprised in the composition the sum of the added amounts shall be inside the mentioned ranges and refers to the total amount of the powder or unit dose composition.

Suitable surfactants for additional use in the composition herein may be nonionic surfactants, gemini surfactants, cationic surfactants, or a combination thereof, wherein nonionic surfactants are particularly preferred.

Suitable nonionic surfactants are alkoxylated fatty alcohols or alkoxylated fatty acids, wherein the alkoxy-chain comprises or essentially consists of less than 15 ethylene oxide, propylene oxide or butylene oxide units or combinations thereof. Preferably said alkoxy-chain comprises or essentially consists of ethylene oxide or propylene oxide units, in particular ethylene oxide units. The length of the alkoxy chain of the additional surfactants is below 15 and preferably ranges from 2 to 14, more preferred from 3 to 10, even more preferred from 3 to 8, including 4, 5, 6, and 7. It is preferred that the chain comprises mainly or consists of ethylene oxide. Ethoxylated glycerol compounds like Glycereth-6 Cocoate are particularly preferred.

The hydrocarbon-chain of the fatty alcohol or fatty acid can be saturated, mono-unsaturated or poly-unsaturated and linear or branched. The length of this hydrocarbon-chain varies between 4 and 30 carbon molecules, including 6, 8, 10, 11, 12, 14, 16, 18, 20, 22, 24, 26 or 28.

Further preferred are nonionic surfactants like alkylglycosides, even more preferred alkylglucosides, which are glucose ethers with a fatty alcohol. Particularly preferred alkylglucosides have a glucose etherified with a C₆₋₂₀ fatty alcohol, e.g. octylglucoside (caprylglucoside), decylglucoside (capringlucoside), dodecylglucoside (laurylglucoside), tetradecylglucoside (myristylglucoside), hexadecylglucoside (cetylglucoside) or octadecylglucoside (stearylglucoside), without being limited to the mentioned. Further, alkyl polyglucosides (APG) may be produced by a reaction of a fatty alcohol with several glucose units. Preferred APGs can have averagely two to averagely four glucose units and has a hydrocarbon-chain that varies from 4 up to 26 carbon moieties, including 4, 6, 8, 10, 12, 14, 16, 18, 20, 22 C. Said alkylglucosides and / or APGs (reference to both types is herein defined as alkyl(poly)glucosides) can be the only type of nonionic surfactants in the composition.

Another type of nonionic surfactants are glucamide surfactants. Glucamides are the reaction product of glucose amine with fatty acid, particularly preferred alkylglucamides have an alkyl chain of C₆₋₂₀ hydrocarbon groups. Further, it is preferred that the glucamides have a C₁₋₃ substituent at the amid nitrogen, preferably methyl or ethyl, most preferred methyl, thus represent an N-C₁₋₃ alkyl glucamide, e.g. octoyl methyl glucamide (caproyl methyl glucamide), decoyl methyl glucamide (caprinoyl methyl glucamide), dodecoyl methyl glucamide (lauroyl methyl glucamide), tetradecoyl methyl glucamide (myristoyl methyl glucamide), hexadecoyl methyl glucamide (cetoyl methyl glucamide) or octadecoyl methyl glucamide (stearoyl methyl glucamide) without being limited to the mentioned e.g. the glucamides offered under the tradename GlucoTain by Clariant, or Cocoyl Methyl Glucamide (INCl) or mixtures thereof.

Further preferred classes of additional surfactants are gemini surfactants. Their properties are dependent of the chosen tail, ion-group and spacer. The tail can for example consists of glucoside, poly-glucoside or a hydrocarbon chain. The hydrocarbon chain can be saturated or unsaturated, branched or linear. The ion-group can be anionic (e.g., phosphate, sulphate, carboxylate) or nonionic. The spacer can be short or long methylene groups, rigid (stilbene), polar (polyether, polyethylene oxide), and nonpolar (aliphatic, aromatic).

Further optional additional surfactants are cationic surfactants. Such surfactants have typically a quaternary nitrogen and usually four alkyl chains independently with C₁ to C₂₀. Examples for the alkyl chains are independently methyl-, ethyl-, propyl-, butyl- as typical short chains and lauryl-, myristyl-, palmityl- or stearyl-chains as typical long chains. A typical cationic surfactant has at least one short chain, often two short chains e.g., methyl, and at least two longer chains, e.g. independently selected from lauryl-, myristyl-, palmityl- or stearyl-chains. If present, it is preferred that cationic surfactants are present in a total amount of below 5 wt.%, preferably below 3 wt.%, e.g. in a range of from 0.1 to 2 wt.% of the total amount of the powder or unit dose detergent composition.

### Conditioning polymer

For use as a personal care composition, the detergent composition may further comprise at least one cationic polymer, in particular a "conditioning polymer" for conditioning the hair and/or skin. Conditioning polymers are known in the art and are represented e.g., by polysaccharides like cellulose and guar polymers and their derivatives, modified by quarternary ammonium compounds.

The cationic polymers may be homopolymers or formed from two or more types of monomers. The molecular weight of the polymer may be between 5,000 and 10,000,000, typically at least 10,000, and preferably in the range 100,000 to about 2,000,000. These polymers will typically have cationic nitrogen containing groups such as quaternary ammonium or protonated amino groups, or a mixture thereof. The cationic charge density is suitably at least 0.1 meq/g, preferably above 0.8 or higher. In some instances, the cationic charge density does not exceed 3 meq/g, or does not exceed 2 meq/g. The charge density can be measured using the Kjeldahl method and can be within the above limits at the desired pH of use, which will in general be from about 3 to 9 and preferably between 4 and 8.

Examples of suitable cationic conditioning polymers are polymers of the Polyquaternium type or those described in EP 150 1873 A, without being limited to the mentioned.

The cationic nitrogen-containing group will generally be present as a substituent on a fraction of the total monomer units of the cationic conditioning polymer. Thus when the polymer is not a homopolymer it can contain further non-cationic monomer units.

The cationic amines can be primary, secondary or tertiary amines, depending upon the particular species and the pH of the composition.

The cationic conditioning polymers can comprise mixtures of monomer units derived from amine- and/or quaternary ammonium-substituted monomer and/or compatible spacer monomers.

Suitable cationic conditioning polymers include, for example: copolymers of 1 -vinyl-2-pyrrolidine and 1 -vinyl-3-methyl-imidazolium salt (e.g., Chloride salt) (referred to as Polyquaternium-16) such as those commercially available from BASF under the LUVIQUAT tradename (e.g., LUVIQUAT FC 370); copolymers of 1 -vinyl-2-pyrrolidine and dimethylaminoethyl methacrylate (referred to as Polyquaternium-11) such as those commercially from Gar Corporation (Wayne, N.J., USA) under the GAFQUAT tradename (e.g., GAFQUAT 755N); and cationic diallyl quaternary ammonium-containing polymer including, for example, dimethyldiallyammonium chloride homopolymer and copolymers of acrylamide and dimethyldiallyammonium chloride (referred to as Polyquaternium-6 and Polyquaternium-7).

Preferred cationic conditioning polymers include polysaccharide polymers, such as cationic cellulose derivatives and cationic starch derivatives. Cationic cellulose is available from Amerchol Corp. (Edison, N.J., USA) in their Polymer JR (trade mark) and LR (trade mark) series of polymers, as salts of hydroxyethyl cellulose reacted with trimethyl ammonium substituted epoxide (referred to as Polyquaternium-10). Another type of cationic cellulose includes the polymeric quaternary ammonium salts of hydroxyethyl cellulose reacted with lauryl dimethyl ammonium-substituted epoxide (referred to as Polyquaternium-24). These materials are available from Amerchol Corp. (Edison, N.J., USA) under the tradename Polymer LM- 200.

Further preferred cationic polymers include cationic guar gum derivatives, such as guar hydroxypropyltrimonium chloride.

Polymers of the Polyquaternium type are known and comprise Polyquaternium-1 (ethanol, 2, 2', 2 ' ' -nitrilotris-, polymer with 1,4-dichloro-2-butene and N,N,N',N'-tetramethyl-2-butene-1,4-diamine), Polyquaternium-2 (poly[bis(2-chloroethyl)ether-1,3-bis[3-(dimethylamino)propyl]urea]), Polyquaternium-4 (hydroxyethyl cellulose dimethyl diallylammonium chloride copolymer; Diallyldimethylammonium chloride-hydroxyethyl cellulose copolymer), Polyquaternium-5 (copolymer of acrylamide and quaternized dimethylammoniumethyl methacrylate), Polyquaternium-6 (poly(diallyldimethylammonium chloride)), Polyquaternium-7 (copolymer of acrylamide and diallyldimethylammonium chloride), Polyquaternium-8 (copolymer of methyl and stearyl dimethylaminoethyl ester of methacrylic acid, quaternized with dimethylsulphate), Polyquaternium-9 (homopolymer of N,N-(dimethylamino)ethyl ester of methacrylic acid, quaternized with bromomethane), Polyquaternium-10 (quaternized hydroxyethyl cellulose), Polyquaternium-11 (copolymer of vinylpyrrolidone and quaternized dimethylaminoethyl methacrylate), Polyquaternium-12 (ethyl methacrylate / abietyl methacrylate / diethylaminoethyl methacrylate copolymer quaternized with dimethyl sulfate), Polyquaternium-13 (ethyl methacrylate / oleyl methacrylate / diethylaminoethyl methacrylate copolymer quaternized with dimethyl sulfate), Polyquaternium-14 (trimethylaminoethylmethacrylate homopolymer), Polyquaternium-15 (acrylamide-dimethylaminoethyl methacrylate methyl chloride copolymer), Polyquaternium-16 (copolymer of vinylpyrrolidone and quaternized vinylimidazole), Polyquaternium-17 (adipic acid, dimethylaminopropylamine and dichloroethylether copolymer), Polyquaternium-18 (azelanic acid, dimethylaminopropylamine and dichloroethylether copolymer), Polyquaternium-19 (copolymer of polyvinyl alcohol and 2,3-epoxypropylamine), Polyquaternium-20 (copolymer of polyvinyl octadecyl ether and 2,3-epoxypropylamine), Polyquaternium-22 (copolymer of acrylic acid and diallyldimethylammonium chloride), Polyquaternium-24 (auaternary ammonium salt of hydroxyethyl cellulose reacted with a lauryl dimethyl ammonium substituted epoxide), Polyquaternium-27 (block copolymer of Polyquaternium- 2 and Polyquaternium-17), Polyquaternium-28 (copolymer of vinylpyrrolidone and methacrylamidopropyl trimethylammonium), Polyquaternium-29 (chitosan modified with propylene oxide and quaternized with epichlorhydrin), Polyquaternium-30 (ethanaminium, N-(carboxymethyl)-N,N-dimethyl-2-[(2-methyl-1 -oxo-2-propen-1 -yl)oxy]-, inner salt, polymer with methyl 2-methyl-2-propenoate), Polyquaternium-31 (N,N- dimethylaminopropyl-N-acrylamidine quaternized with diethylsulfate bound to a block of polyacrylonitrile), Polyquaternium-32 (poly(acrylamide 2-methacryloxyethyltrimethyl ammonium chloride)), Polyquaternium-33 (copolymer of trimethylaminoethylacrylate salt and acrylamide), Polyquaternium-34 (copolymer of 1,3-dibromopropane and N, N-diethyl- N',N'-dimethyl-1,3-propanediamine), Polyquaternium-35 (methosulphate of the copolymer of methacryloyloxyethyltrimethyl-ammonium and of methacryloyloxyethyldimethylacetylammonium), Polyquaternium-36 (copolymer of N,N- dimethylaminoethylmethacrylate and buthylmethacrylate, quaternized with dimethylsulphate), Polyquaternium-37 (poly(2-methacryloxyethyltrimethylammonium chloride)), Polyquaternium-39 (terpolymer of acrylic acid, acrylamide and diallyldimethylammonium Chloride), Polyquaternium-42 (poly[oxyethylene(dimethylimino)-ethylene (dimethylimino)ethylene dichloride]), Polyquaternium-43 (copolymer of acrylamide, acrylamidopropyltrimonium chloride, 2- amidopropylacrylamide sulfonate and dimethylaminopropylamine), Polyquaternium-44 (3- Methyl-1 -vinylimidazolium methyl sulfate-N-vinylpyrrolidone copolymer), Polyquaternium- 45 (copolymer of (N-methyl-N-ethoxyglycine)methacrylate and N,N- dimethylaminoethylmethacrylate, quaternized with dimethyl sulphate), Polyquaternium-46 (terpolymer of vinylcaprolactam, vinylpyrrolidone, and quaternized vinylimidazole), and Polyquaternium-47 (terpolymer of acrylic acid, methacrylamidopropyl trimethylammonium chloride, and methyl acrylate).

Preferably the personal care compositions like e.g. hair treatment compositions include one or more of such cationic polymers selected from cationic cellulose derivatives, quaternized hydroxyethyl cellulose (e.g., polyquaternium-10), cationic starch derivatives, cationic guar gum derivatives, copolymers of acrylamide and dimethyldiallyammonium chloride (e.g., polyquaternium-7), or a mixture thereof. It is preferred that at least one of the cationic polymer(s) is selected from Polyquaterniums, for example, Polyquaternium-4, Polyquaternium-5, Polyquaternium-6, Polyquaternium-7, Polyquaternium-10, Polyquaternium-11, Polyquaternium-16, Polyquaternium-22, Polyquaternium-24, Polyquaternium-37, Polyquaternium-39, Polyquaternium-44, Polyquaternium-47, Polyquaternium-53, Polyquaternium-67, Polyquaternium-72, and a mixture thereof. Preferred Polyquaternium polymers are Polyquaternium 6, 7, 10, 11, 16, 24 and 44. Further preferred, the polyquaterniums are selected from Polyquaternium-7, Polyquaternium-10, and a mixture thereof.

In some embodiments, the cationic polymers are selected from cationic polysaccharides, for example, cationic polysaccharides selected from cationic cellulose derivatives, cationic starch derivatives, cationic guar derivatives, cationic locust bean gum derivatives, and a mixture thereof. In some cases, the hair treatment compositions preferably include at last one cationic cellulose derivative selected from Polyquaternium-4, Polyquaternium-10, Polyquaternium-24, Polyquaternium-67 and/or Polyquaternium-72, and a mixture thereof.

Particularly preferred cationic polymers are guar hydroxypropyltrimonium chloride and Polyquaternium 10. The presence of any of those two, in particular the presence of Polyquaternium-10 is particularly preferred.

The total amount of cationic polymer(s) in the ready-made liquid or jelly personal care composition, if present, can vary but is typically about 0.01 to about 10 wt.%, based on the total weight of the personal care composition. In some instances, the total amount of the cationic polymer(s) is about 0.01 to about 5 wt.%, about 0.01 to about 3 wt.%, about 0.01 to about 2 wt.%, about 0.01 to about 1 wt.%, about 0.05 to about 5 wt.%, about 0.1 to about 4 wt.%, about 0.1 to about 3 wt.%, about 0.1 to about 2 wt.%, about 0.1 to about 1 wt.%, about 0.1 to about 0.5 wt.%, including all ranges and subranges therebetween.

Accordingly, if present in the powder or unit dose composition of the invention, the cationic polymer preferably is provided in an amount allowing to provide said polymer in the above mentioned ranges if dissolved/diluted in water, thus, in an amount of up to 50 wt.%, whereas it is preferred that the powder or unit dose composition comprises such polymer(s) in an amount of 0.1 to 30 wt.-%. Preferably the total amount of conditioning polymer(s) in a personal care composition according to the invention is in the range of from 0.1 to 20 wt.%, 0.5 to 20 wt.%, or 1 to 20 wt.%, or 1 to 10 wt.%, or even 1 to 5 wt.%, wherein said amounts can be provided by one of the conditioning polymers, but in case that more than one such polymers are contained, the maximum amount as defined above is not exceeded.

### pH adjusting agents

The pH of the ready-prepared liquid or jelly personal care or household care composition can be in the range of pH 3 to 9, e.g. pH 3.5 to 8. The preferred pH of the liquid or jelly personal care or household care composition in the range of pH 3.8 to 7, more preferably pH 4 to 6 or pH 4 to 5.5 or even pH 4 to pH 5 and may directly result from the ratio of the acidic and basic components of the organic builder system. Nonetheless, it might be further suitable to adapt the pH of the solution by adding further pH adjusting agents to the powder or unit dose composition.

By "acidifier/acidification agents" herein it is meant any component which when released, acts such as to reduce the pH of the obtained solution containing the dissolved/dispersed composition. Suitable acidification agents for use in the composition of the present invention include inorganic and organic acids including, for example, carboxylate acids except citric acid, like e.g., succinic acids, tartaric acid, oxalic acid, polylactic acid or polyitaconic acid, and also acetic acid, boric acid, malonic acid, adipic acid, fumaric acid, lactic acid, glycolic acid, maleic acid, their derivatives and any mixtures of the foregoing.

Alkaline agents for raising the pH up to 7, 8 or even 9 might be selected from soap, NaOH and KOH.

The pH adjusting agents are added in an amount sufficient to reach the desired pH in the solution prepared by dissolving the powder or unit dose composition according to the invention in water as described above.

### Perfumes

Suitable perfumes include blooming perfumes, perfume oils, and perfume raw materials comprising alcohols, ketones, aldehydes, esters, ethers, nitriles alkenes, and mixtures thereof. Examples of ester type perfumes include benzylacetate, phenoxyethylisobutyrat, p-tert.-butylcyclohexylacetat, linalylacetat, dimethylbenzyl-carbinylacetate, phenylethyl acetate, linalyl benzoate, benzylformiate, ethylmethylphenyl glycinate, allylcyclohexyl propionate, styrallyl propionats and benzylsalicylate. Examples for ether type perfumes are benzylethylether, and for example linear C₈-18 alkanals, citral, citronellal, citronellyl oxyacetaldehyde, cyclamenaldehyde, hydroxycitronellal, lilial and bourgeonal, examples for ketones are ionones, alpha -isomethylionone and methyl-cedrylketone, examples for alcohols are anethol, citronellol, eugenol, geraniol, linalool, phenylethylalkohol and terpineol, examples for carbohydrates are terpene including limonen and pinen. Mixtures of different perfumes generating distinct fragrances may be preferred. These may include naturally occurring oils such as for example from pine, citrus, jasmine, patchouli, rose, ylang ylang, sage, camomille, cloves, balm, mint, orange flower, orange peel, sandel wood, neroliol, cinnamon, lime-tree blossom, juniper berry, vetiver, olibanum, galbanum and labdanum.

If present, the perfumes may preferably be present in an amount of up to 3 wt.-%, more preferably in an amount of from 0.1 to 2.5 wt.-% or from 0.2 to 2 wt.-%, based on the total weight of the composition.

### Colorants/dyes

Suitable colorants and dyes include all kinds of colorants and dyes Particularly suitable are colorants which are water soluble or soluble in at ambient temperature liquid organic solvents.

Further suitable examples of colorants include anionic dyes like for example anionic nitroso dyes. One possible suitable dye is naphthol green (Colour Index (CI) part 1: Acid Green 1; Teil 2: 10020), which is commercially available as Basacid TM Green 970 from BASF, (Ludwigshafen, Germany) and mixtures thereof with suitable blue dyes. Further suitable colorants are for example Pigmosol TM Blue 6900 (CI 74160), Pigmosol TM Green 8730 (CI 74260), Basonyl TM Red 545 FL (CI 45170), Sandolan TM Rhodamin EB400 (CI 45100), Basacid TM Yellow 094 (CI 47005), Sicovit TM Patentblue 85 E 131 (CI 42051), Acid Blue 183 (CAS 12217-22-0, CI Acidblue 183), Pigment Blue 15 (CI 74160), Supranol TM Blue GLW (CAS 12219-32- 8, CI Acidblue 221), Nylosan TM Yellow N-7GL SGR (CAS 61814-57-1, CI Acidyellow 218) und/oder Sandolan TM Blue (CI Acid Blue 182, CAS 12219-26-0).

If present, the amount of colorants is preferably from 0.01 to below 1 wt.-%, preferably below 0.5 wt.-%, more preferably below 0.3 wt.-%.

### Preservatives

Suitable preservatives for use herein include compounds with antimicrobial effect. If present, suitable preservatives according to the present invention may be selected from the group including alcohols, aldehydes, antimicrobial acids, carbonic acid esters, acid amides, phenols, phenol derivatives, sorbates like sodium or potassium sorbate, diphenyls, diphenyl alkans, urea derivatives, oxygen-nitrogen-acetales, formates, benzamidines, substituted isothiazoles, and hydrated isothiazol derivatives, such as for example isothiazolinen and isothiazolidinen, phthalimid derivatives, pyridine derivatives, antimicrobial surfactants, such as for example antimicrobial quarternary surfactants, guanidines, antimicrobial amphoteric compounds, chinoline, 1,2-dibrom-2,4-dicyanobutane, iodo-2-propynyl-butyl-carbamate, iodine, iodophore and peroxide including for example phenoxyethanol, undecylene acid, salicylic acid, benzoeic acid or their sodium or potassium salts, in particular sodium benzoate, 2-benzyl-4-chlorphenol, 2,2'-methylen-bis-(6-brom-4-chlorphenol), 2,4,4'-trichlor-2'-hydroxydiphenylether, N-(3-aminopropyl)-N-dodecylpropame-1,3-diamine, N-(4-chlorphenyl)-N- (3,4-dichlor-phenyl)-urea, N,N'-(1,10-decandiyldi-1-pyridinyl-4-yliden)-bis-(1-octanamin)-dihydro-chlorid and N,N'-bis-(4-chlorphenyl)-3,12-diimino-2,4,11,13-tetraaza-tetradecandiimid-amide, as for example disclosed in H. Wailhäusser "Praxis der Sterilisation, Desinfektion, Konservierung, Keimidentifizierung, Betriebshygiene" (5^{th} edition, Stuttgart; New York: Thieme, 1995.

Particularly preferred preservatives are potassium sorbate and/or sodium benzoate. The preservative(s) are present in a suitable amount, preferably in a total amount of preservatives of from 0.5 to 7 wt.-%, more preferred 1 to 6 wt.-% or 2 to 6 wt.-% of the total weight of the powder or unit dose composition.

### Natural oils and fats

Optionally, depending on the intended application, the personal care or household care composition according to the current invention may include one or more natural oils and fats such as soy bean oil, rice barn oil, jojoba oil, avocado oil, almond oil, olive oil, cacao butter, sesame oil, parsic oil, castor oil, coconut oil, mink oil, beef tallow and lard: hardened oils obtained by hydrogenating these natural oils and fats and synthesized triglycerides such as glyceride myristate and 2-ethylhexanoic glyceride.

If present, the amount of natural oils and fats ranges preferably from 0.01 to 20 wt.-%, preferably from 0.5 wt.-% to 15 wt.-%, more preferably below 10 wt.-%.

### Mineral salts

It is particularly preferred that the powder or unit dose composition is essentially free of Ca and Mg ions, thus, doesn't comprise any component including Ca or Mg, wherein "essentially free" means that the composition comprises less than 1% of such ions, preferably less than 0.5%, more preferred less than 0.2%, less than 0.1% or less than 0.05%. Most preferred the water softener composition is free of such ions.

However, in some embodiments, e.g., if the current invention is used as a bath additive, it is known that mineral salts have a circulation-promoting, disinfecting and anti-inflammatory effect. Especially salts of the cations sodium, potassium, magnesium, calcium, iron, ammonium or manganese and the anions hydrogen carbonate, chloride, fluoride, sulfate or nitrate can be present individually or in mixtures.

If present, the amount of mineral salts ranges preferably from 1 to 20 wt.%, preferably from 1.5 wt.% to 15 wt.%, more preferably the mineral salt content is below 10 wt.-%.

### Humectants and Emollients

Optionally, to further improve the beneficial effect of the personal care or household care composition according to the current invention to the skin, emollients and/or humectants can be added.

Representative humectants include glycerol, polyethylene glycol, polyvinyl alcohol, sorbitol, polyvinylpyrollidone glycerol, sorbitol, diglycerol, triglycerol, polyglycerol, erythritol, pentaerythritol, glucose, galactose, fructose, sucrose, maltose, xylose, xylobiose, reduced oligosaccharides, etc. Emollients may include mineral oils, fatty acid esters, fatty alcohols, dimethicones, etc. Especially preferred as a humectant is glycerol.

If present, the amount of humectants and emollients ranges preferably from 0.01 to 15 wt.-%, preferably from 0.5 to 10 wt.-%, most preferred from 0.8 to below 8 wt.-%, e.g. 7, 6 or 5 wt.%.

### Kit of use: powder / unit dose and container for the liquid or jelly composition

The present invention further refers to a kit comprising at least one container for preparing the liquid or jelly personal care or household care composition therein by adding a specified volume of water to a weighed amount of powder or a unit dose.

Said kit comprises at least one sachet, pouch or unit dose consisting of a weighted amount of powder of the composition and a container. The kit preferably comprises at least 2, more preferred at least 3, at least 5, at least 8 or at least 10 sachets, pouches or unit doses of the composition of the present invention and a container.

Said container preferably is adapted to the intended use, e.g., can be provided with a pull-to open cap, a push-to-open cap, a turn-to-open cap, a spray head, a dosing system etc. Further, the container provides an opening designed concerning size and shape so that the powder or unit dose can be fed through and/or included into the container without any difficulties. Preferably the container comprises the opening at the top, which after addition of the powder or unit dose is closed with the cap or head suitable for use.

Since the container is re-usable for numerous uses, the powder or unit dose(s) for further use can also be provided separately. The powder or unit dose may be provided in any suitable package.

According to the invention it is particularly preferred that all the main ingredients representing at least 5 wt% of the total amount, preferably at least 8 wt% of the total composition are added as solid compounds: Thus; the composition of the invention is formed mainly by solid ingredients (at least 90 %, preferably at least 92 %, more preferred at least 95 % of the total composition is formed by components which are solid at 25 °C). If liquid ingredients like e.g., perfumes or liquid surfactants have to be added, they are applied on any solid component of the composition, e.g. by spraying.

### Use/application of the liquid or jelly composition

The transparent liquid or jelly composition prepared by addition of the powder / unit dose of the present invention to water is intended for use as a personal care or household care composition, in particular for cleaning hard surfaces, e.g., as a dishwashing agent, in particular for hand dishwashing, for hand wash laundry, for bath or floor cleaning or for any other household application, in particular where the liquid or jelly composition comes in contact with the user's skin.

Another application is for personal care, e.g., as a shampoo, shower gel, face cleaner, hand cleaner, cleaner for genital area, body scrub, hair conditioner or bath additive or for any other cosmetic composition.

The transparent liquid or jelly composition is preferably prepared by adding the dosed amount of the composition to the specified amount of 30 to 40 °C warm water, dissolving the composition by gentle swinging or pivoting and then allowing the liquid/jelly composition to cool to room temperature (20 to 25 °C).

### EXAMPLE

A powder for personal care or household care compositions has been provided having the composition as shown in table 1 and 2. All ingredients have been added as solid components. If a small amount of a liquid component had to be added, it was sprayed on a solid component. The transparent liquid or jelly personal care or household care composition has been prepared by adding 25 g of the powder into 225 ml of water with a temperature of 35 °C.

**Table 1: Formulation of the composition for preparing a household care composition**

| INCI | Amount [wt.%] |
|---|---|
| Citric acid | 10 |
| MGDA | 5 |
| Sodium citrate | 27 |
| Sodium lauryl sulfate | 35 |
| Coco amidopropyl betaine | 12 |
| Sodium chloride; preservative; nonionic surfactant, miscellaneous | Ad 100 |

The solid composition did not comprise any thickening polymeric compound and no added water.

**Table 2: Example formulations of the composition for preparing a personal care composition**

| INCI | Shampoo | Amount [wt.%] Shower gel | Hand cleaner |
|---|---|---|---|
| Citric acid | 9,95 | 9,95 | 9,95 |
| MGDA | 4,05 | 4,05 | 4,05 |
| Sodium citrate | 20 - 24 | 20 - 24 | 20 - 24 |
| Sodium coco sulfate | 32,2 | 32,2 | 32,2 |
| Coco amidopropyl betaine | 11,94 | 11,94 | 11,94 |
| Guar hydroxypropyltrimonium chloride | 0,9 | 0,9 | - |
| Sodium chloride; preservatives; nonionic surfactant, miscellaneous | Ad 100 | Ad 100 | Ad 100 |

## Claims

1. A solid detergent composition in form of a powder or unit dose for preparing a ready-to-use personal care or household care detergent composition, said powder or unit dose comprising:
(i) at least one amphoteric surfactant, wherein the amphoteric surfactant(s) is/are present in an amount ranging from a total amount of from 1 to 40 wt.-%, more preferred in an amount of from 3 to 35 wt.-%, even more preferred from 5 to 30 wt.-%, 8 to 25 wt.-% or 10 to 20 wt.-% based on the total weight of the powder or unit dose, and
(ii) at least one anionic surfactant, and
(iii) less than 5 wt-% of a polymeric thickening agent, selected from non-charged polysaccharides and polymers comprising acrylic monomers.

2. The powder or unit dose according to any of the preceding claims, wherein the anionic surfactant(s) (ii) is/are present in a total amount of from 5 to 60 wt.-%, preferably in an amount of from 10 to 55 wt.-%, more preferred in an amount of from 13 to 52 wt.-%, even more preferred from 15 to 50 wt.-% of the total weight of the powder or unit dose.

3. The powder or unit dose according to any of the preceding claims, wherein the ratio of amphoteric to anionic surfactant preferably ranges from 4:1 to 1:12,5, more preferably from 3:1 to 1:10, even more preferably from 2:1 to 1:8, most preferably from 1:1 to 1:5 (wt/wt).

4. The powder or unit dose according to any of the preceding claims, wherein at least one of the amphoteric surfactant(s) (i) is/are selected from betaines or sulphobetaines.

5. The powder or unit dose according to any of the preceding claims, wherein at least one of the anionic surfactant(s) (ii) is/are selected from alkyl sulphates or sulphonates.

6. The powder or unit dose according to any of the preceding claims, further comprising a builder system, preferably in a total amount of from 15 to 60 wt.-%, preferably from 20 to 55 wt.-%, more preferred from 22 to 53 wt.-%, even more preferred from 25 to 50 wt.-% of the composition.

7. The powder or unit dose according to claim 6, wherein said builder system comprises citric acid and citrate, which preferably represent together at least 60 wt.-% of the builder system.

8. The powder or unit dose according to any of the preceding claims, further comprising a chelating agent, preferably in a total amount of from 0.1 to 15 wt.-%, preferably from 0.3 to 10 wt.-%, more preferred from 0.5 to 8 wt.-%, even more preferred from 0.8 to 5 wt.-% or 1 to 3 wt.-% of the composition.

9. The powder or unit dose according to claim 8, wherein the chelating agent is selected from any biodegradable chelating agent, preferably from MGDA, GLDA or ethylene diamine N, N'-disuccinic acid, in particular their alkali metal, alkaline earth, or ammonium salts, preferably their sodium salts; or from phosphonates.

10. The powder or unit dose according to any of the preceding claims, wherein the powder or unit dose comprises at least one further ingredient selected from further surfactants, preservatives, colorants, dyes and/or perfumes.

11. The powder or unit dose according to any of the preceding claims, wherein the powder / unit dose
(a) comprises at least one preservative, and / or
(b) has an amount of less than 5 wt.-% nonionic surfactant, and / or
(c) is free of phosphates, and / or
(d) is free of any acrylates, and / or
(e) is free of any alkyl benzene sulfonate, and/or
(f) results in a pH in the range of 3 to 9, preferably pH 3.5 to 8, or pH 3.8 to 7, more preferably pH 4 to 6 or pH 4 to 5.5 or even pH 4 to pH 5 when dissolved with water in the range of 1:8 to 1:12 (detergent:water), and/or
(g) comprises a cationic conditioning polymer.

12. A unit dose, represented by a sachet, package or pouch containing a pre-dosed amount of the powder as defined by any of the preceding claims, or a tablet prepared of a pre-dosed amount of the composition as defined by any of the preceding claims.

13. Use of a powder or unit dose as defined in any of the preceding claims for preparing a liquid or jelly personal care or household care detergent composition, by adding a weighed amount of powder or a unit dose to a specified volume of water, preferably in the range 1:8 to 1:12 (detergent:water), said detergent composition having a pH in the range of 3 to 9, preferably pH 3.5 to 8, or pH 3.8 to 7, more preferably pH 4 to 6 or pH 4 to 5.5 or even pH 4 to pH 5.

14. A liquid or jelly detergent composition prepared by addition of a powder or unit dose of a detergent composition as defined in any of the preceding claims into a specified volume of water, preferably in a ratio of from 1:8 to 1:12 (detergent:water) , wherein said detergent composition is suitable as a household care composition or a personal care composition coming into contact with the users skin.

15. A kit for preparing a liquid or jelly personal care or household care detergent composition comprising the powder or unit dose as defined in claims 1 to 12 and a container having an opening shaped and sized to feed said tablet or unit dose through said opening and a cap or head for closing said opening of the container.
